# EUROPEAN PATENT APPLICATION

(11) **EP 3 958 275 A1**
(43) Date of publication of application: **23.02.2022**
(21) Application number: 21192254.7
(22) Date of filing: 19.08.2021
(51) Int. Cl.: G16H 50/20, G16H 50/50, A61B 5/00

(54) **SYSTEMS, METHODS AND DEVICES FOR SLEEP INTERVENTION QUALITY ESTIMATION**

(30) Priority: 21.08.2020 US 202017000218
(71) Applicant: StimScience Inc., Berkeley, CA 94710 (US)
(72) Inventor: Gurumoorthy, Ram, Berkeley, 94710 (US)
(74) Representative: Hepworth Browne

(57) **Abstract**

Provided are systems, methods, and devices for sleep intervention quality estimation. Systems include a communications interface configured to receive measurement data from a plurality of data sources, the measurement data comprising a plurality of measurements of biological parameters of a user before and after a sleep intervention treatment, the communications interface being further configured to receive treatment data comprising one or more treatment parameters associated with the sleep intervention treatment. Systems also include a processing device comprising one or more processors configured to generate at least one sleep model for the user based on the received measurement data and treatment data, the processing device being further configured to generate a user interface based, at least in part, on the at least one sleep model, and a memory device configured to store the at least one sleep model.

## Description

### TECHNICAL FIELD

The present disclosure relates to mechanisms and processes directed to measurements of brain activity and sleep intervention quality estimation.

### BACKGROUND

Human sleep can be measured using several aspects of the human physiology including their brain activity, their heart activity, their eye activity, temperature, movement, oxygen saturation, and the like. A human brain may include neurons which exhibit measurable electrical signals when active. Accordingly, various measuring modalities, such as electrodes, may be used to measure such electrical activity. The neural activity of neurons may include many a variety of frequency components. Accordingly, such electrical activity may be measured and represented as a power spectrum in a frequency domain. Moreover, such measurements may be obtained as a user sleeps. Similarly, other measurements may be obtained, such as heart rate activity that includes a heart rate (mean, minimum or maximum over a period, mean square over a period), as well as heart rate variability (beat-to-beat, or beat-to-beat aggregated over a window of time). However, traditional techniques for measuring such electrical activity in such contexts remain limited in their ability to utilize such measurements, and more specifically, to efficiently and effectively predict the efficacy of the implementation of different sleep intervention strategies and techniques.

### SUMMARY

Provided are systems, methods, and devices for sleep intervention quality estimation. Systems include a communications interface configured to receive measurement data from a plurality of data sources, the measurement data comprising a plurality of measurements of biological parameters of a user before and after a sleep intervention treatment, the communications interface being further configured to receive treatment data comprising one or more treatment parameters associated with the sleep intervention treatment, a processing device comprising one or more processors configured to generate at least one sleep model for the user based on the received measurement data and treatment data, the processing device being further configured to generate a user interface based, at least in part, on the at least one sleep model, and a memory device configured to store the at least one sleep model.

In various embodiments, the user interface is configured to display an output to the user, and receive an input form the user via one or more data fields. In some embodiments, the processing device is further configured to receive a plurality of inputs from the user, and generate a plurality of estimated sleep intervention quality metrics based, at least in part, on the received plurality of inputs and the at least one sleep model. In some embodiments, the plurality of estimated sleep intervention quality metrics comprises a representation of a difference between a generated output of the at least one sleep model and a plurality of ideal sleep parameters for the user. According to various embodiments, the processing device is further configured to generate a result object based, at least in part, on the plurality of estimated sleep intervention quality metrics. In various embodiments, the processing device is further configured to display the result object in the user interface. In some embodiments, the processing device is further configured to modify the result object based on an additional input received via the user interface. According to various embodiments, the additional input comprises a change to one or more proposed treatment parameters.

Devices are also disclosed herein that include a communications interface configured to receive measurement data from a plurality of data sources, the measurement data comprising a plurality of measurements of biological parameters of a user before and after a sleep intervention treatment, the communications interface being further configured to receive treatment data comprising one or more treatment parameters associated with the sleep intervention treatment, and a processing device comprising one or more processors configured to generate at least one sleep model for the user based on the received measurement data and treatment data, the processing device being further configured to generate a user interface based, at least in part, on the at least one sleep model.

In various embodiments, the user interface is configured to display an output to the user, and receive an input form the user via one or more data fields. In some embodiments, the processing device is further configured to receive a plurality of inputs from the user, and generate a plurality of estimated sleep intervention quality metrics based, at least in part, on the received plurality of inputs and the at least one sleep model. According to various embodiments, the plurality of estimated sleep intervention quality metrics comprises a representation of a difference between a generated output of the at least one sleep model and a plurality of ideal sleep parameters for the user. In various embodiments, the processing device is further configured to generate a result object based, at least in part, on the plurality of estimated sleep intervention quality metrics. In some embodiments, the processing device is further configured to display the result object in the user interface. According to various embodiments, the processing device is further configured to modify the result object based on an additional input received via the user interface.

This and other embodiments are described further below with reference to the figures.

### BRIEF DESCRIPTION OF THE DRAWINGS

Figure 1 illustrates an example of a system for sleep intervention quality estimation, configured in accordance with some embodiments.
Figure 2 illustrates another example of a system for sleep intervention quality estimation, configured in accordance with some embodiments.
Figure 3 illustrates an example of a flow chart of a method for sleep intervention quality estimation, implemented in accordance with some embodiments.
Figure 4 illustrates another example of a flow chart of a method for sleep intervention quality estimation, implemented in accordance with some embodiments.
Figure 5 illustrates an additional example of a flow chart of a method for sleep intervention quality estimation, implemented in accordance with some embodiments.
Figure 6 illustrates an example of a processing device that can be used with various embodiments.

### DETAILED DESCRIPTION OF EXAMPLE EMBODIMENTS

Reference will now be made in detail to some specific examples including the best modes contemplated by the inventors. Examples of these specific embodiments are illustrated in the accompanying drawings. While the present disclosure is described in conjunction with these specific embodiments, it will be understood that it is not intended to limit the disclosure to the described embodiments. On the contrary, it is intended to cover alternatives, modifications, and equivalents as may be included within the spirit and scope of the disclosure as defined by the appended claims. In addition, although many of the components and processes are described below in the singular for convenience, it will be appreciated by one of skill in the art that multiple components and repeated processes can also be used to practice the techniques of the present disclosure.

In the following description, numerous specific details are set forth in order to provide a thorough understanding of the present invention. Particular embodiments may be implemented without some or all of these specific details. In other instances, well known process operations have not been described in detail in order not to unnecessarily obscure the disclosure.

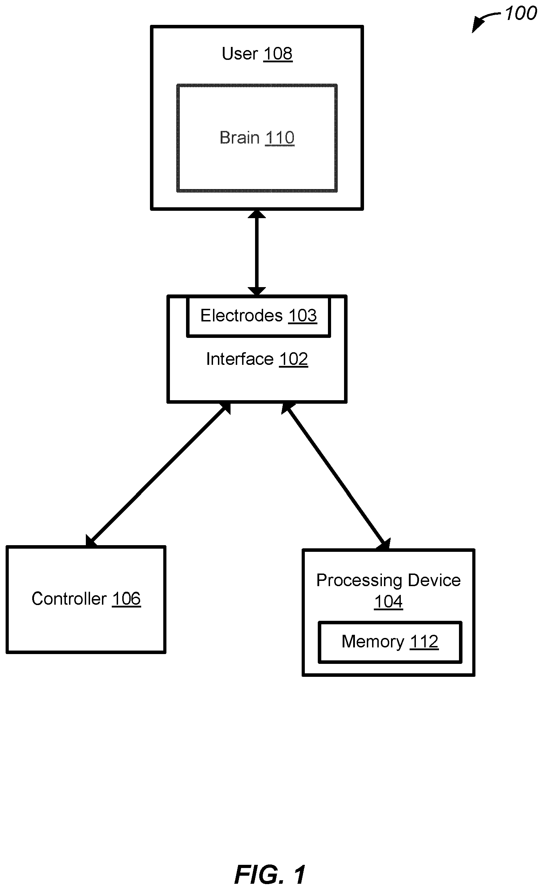

Figure 1 illustrates an example of a system for sleep intervention quality estimation, configured in accordance with some embodiments. As will be discussed in greater detail below, a user may undergo sleep treatments that are intended to enhance and improve a user's sleep. Embodiments disclosed herein enable the identification and generation of sleep quality metrics and models that enable the prediction or estimation of an efficacy of particular treatment modalities for a user.

As will be discussed in greater detail below, components of system 100 may be implemented to estimate the efficacy of sleep treatments of a user, such as user 108. As shown in Figure 1, user 108 may be a person, and may be coupled to components of system 100. More specifically, brain 110 of user 108 may be coupled to system 100 such that system 100 is able to monitor and measure neural activity within brain 110. In some embodiments, the activity is electrical activity that is measured and recorded as electrical measurements. In this way, activity within brain 110 may be monitored during a period of sleep. As will also be discussed in greater detail below, the coupling between user 108 and system 100 may also enable stimulation of neurons within brain 110. Accordingly, system 100 may also modify neural activity of user 108.

In various embodiments, coupling between user 108 and system 100 may be implemented, at least in part, via an interface, such as interface 102. In one example, interface 102 includes a plurality of electrodes. More specifically, such electrodes may eb implemented as an electrode array. Such electrodes may be included in a scalp potential electroencephalogram (EEG) array, may be deep brain stimulation (DBS) electrodes such as electrodes used with intracranial electroencephalography, or may be an epidural grid of electrodes. In other examples, the electrodes may include optogenetics mechanisms for monitoring various neuronal processes or blood saturation. Mechanisms may be used to make various measurements and acquire measurement signals corresponding to neural activity, heart activity, temperature, body/head/eye movements. As used herein, neural activity may refer to spiking or non-spiking activity/potentiation. Moreover, heart activity may be a measure of beat rate or beat-to-beat variability. Furthermore, eye movements may include micro and macro saccades, as well as slow and rapid eye movements.

In various embodiments, such measured signals may be electrical signals derived based on neural activity that may occur in cortical tissue of a brain or may include electrical and optical signals derived from the peripheral parts of the user. Such measurements may be acquired and represented in a time domain and/or frequency domain. In this way, activity may be monitored and measured over one or more temporal windows, and such measurements may be stored and utilized by system 100. In various embodiments, such neural activity may be observed for particular regions of cortical tissue determined, at least in part, based on a configuration of interface 102. In one example, this may be determined based on a configuration and location of electrodes included in interface 102 and coupled with the brain.

According to some embodiments, one or more components of interface 102 are configured to provide stimuli to the brain coupled with interface 102. For example, one or more electrodes included in interface 102 may be configured to provide electrical stimuli to cortical tissue of the brain. As discussed above, such electrodes may be implemented utilizing one or more of various modalities which may be placed on a user's scalp, or implanted in the user's brain.

As will be discussed in greater detail below, such actuation and stimuli provided by interface 102 may be of many different modalities. For example, stimuli may be aural, visual, and/or tactile as well as being electrical and/or magnetic, or any suitable combination of these. Accordingly, interface 102 may further includes additional components, such as speakers, lights, display screens, and mechanical actuators that are configured to provide one or more of aural, visual, and/or tactile stimuli to a user. In this way, any suitable combination of different modalities may be used. For example, a combination of electrical and aural stimuli may be provided via interface 102. Further still, interface 102 may include different portions corresponding to signal acquisition and stimuli administration. For example, a first portion of interface 102 may include electrodes configured to measure neural activity, while a second portion of interface 102 includes speakers configured to generate aural stimuli. In another example, a third portion of interface 102 may include electrodes to measure ECG or heart rate, while a fourth portion may include sensors to measure oxygen saturation.

In some embodiments, interface 102 further includes one or more dedicated processors and an associated memory configured to obtain and store the measurements acquired at interface 102. In this way, such measurements may be stored and made available to other system components which may be communicatively coupled with interface 102.

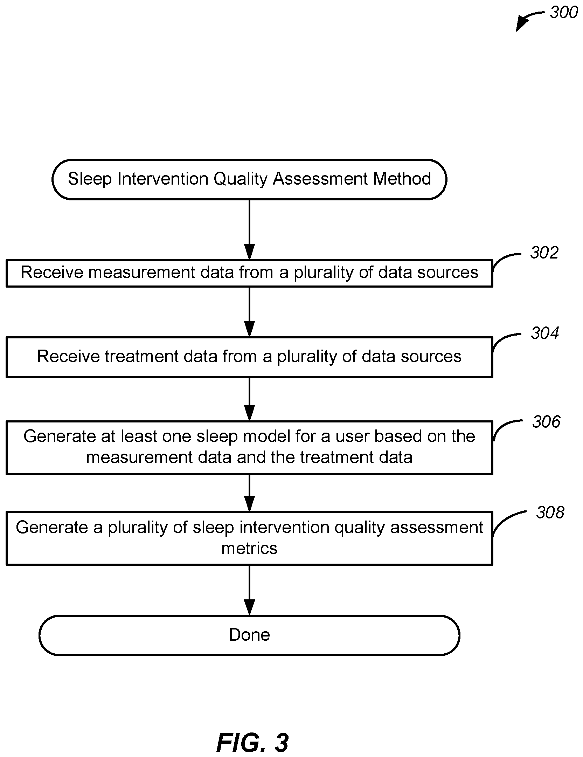

System 100 further includes processing device 104 which may be configured to receive measurements made by interface 102, and may be further configured to generate one or more sleep models that are configured to predict or estimate an efficacy of sleep treatments applied to user 108. As will be discussed in greater detail below, the estimation of the efficacy of sleep treatments may be made based on received measurement data and treatment data which may be used to generate a plurality of quality metrics and a sleep model. Accordingly, processing device 104 is configured to retrieve measurement data from one or more data sources, which may be a memory device or a database system, and is further configured to retrieve measurement data obtained from the user. As will be discussed in greater detail below with reference to Figures 3, 4, and 5, processing device 104 is further configured to generate at least one sleep model based on the received measurement data and treatment data. More specifically, measurement data and treatment data may be used as training data to generate the sleep model. Moreover, the sleep model may be configured to receive inputs, such as a proposed treatment, and generate outputs, such as estimated measurement data and sleep intervention quality estimation metrics.

As will also be discussed in greater detail below, processing device 104 is further configured to generate a result object, such as a report, that provides a summary of the sleep intervention quality assessment metrics. In various embodiments, the report is included in a data object capable of being displayed in a user interface screen. In some embodiments, processing device 104 is further configured to generate a user interface, such as a control panel, that is configured to display an output to a user, and receive an input form the user via one or more data fields. In this way, the user may be provided with a report which may be configurable via the user interface. As will also be discussed in greater detail below, processing device 104 may be further configured to generate one or more recommendations associated with the treatment or intervention strategy, and such recommendations may be included in the user interface screen. In some embodiments, processing device 104 includes memory device 112 which is configured to store quality assessment metrics and result objects, such as reports, generated by processing device 104.

In some embodiments, system 100 includes controller 106 which is configured to generate one or more control signals for interface 102, and is also configured to receive measurements from interface 102. Accordingly, controller 106 may be configured to implement and control the application of one or more sleep treatment modalities. In various embodiments, controller 106 is communicatively coupled with interface 102, and processing device 104. Accordingly, controller 106 is configured to received inputs from various other system components, and generate signals provided to interface 102 based, at least in part on such inputs. As will be discussed in greater detail below, such outputs may be used to provide actuations to the brain coupled with interface 102. For example, outputs generated by controller 106 may be used to stimulate the brain via one or more components of interface 102. In this way, controller 106 may provide stimuli to the brain via interface 102, may receive sleep information via other components such as processing device 104, and may generate stimuli based on such received information.

In some embodiments, controller 106 is configured to implement combined control of pharmacological and stimulation inputs. Accordingly, controller 106 may be configured to modify stimulation inputs based on an expected effect of one or more pharmacological agents that may be administered in conjunction with the stimulation. In this way, controller 106 may modify and control administration of stimuli via interface 102 based on an identified pharmacological regimen. In various embodiments, controller 106 is optionally included in system 100. For example, system 100 might not include controller 106, and such generation of control signals and receiving of measurements may be implemented by processing device 104.

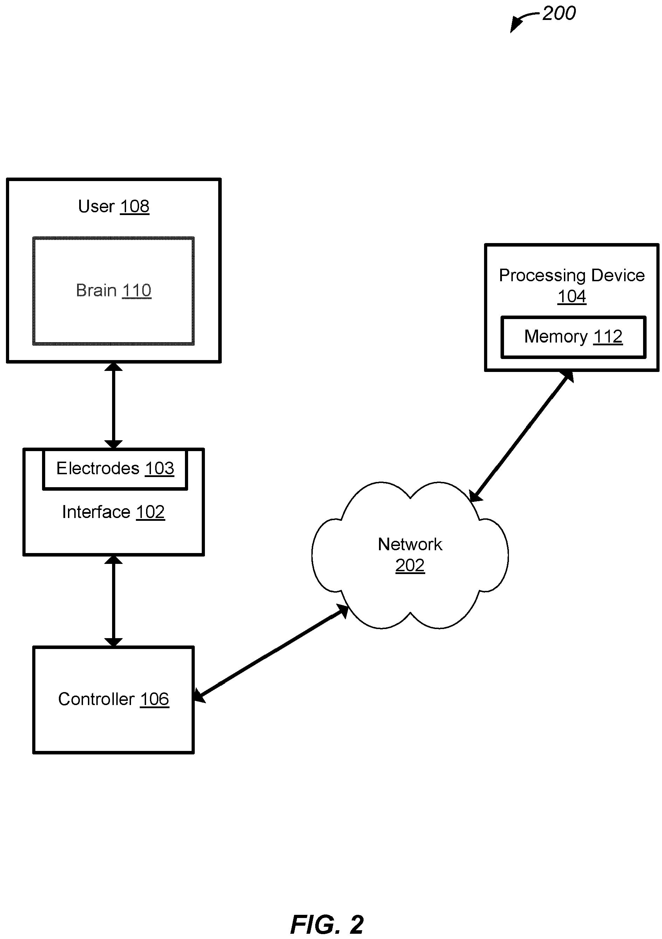

Figure 2 illustrates another example of a system for sleep intervention quality estimation, configured in accordance with some embodiments. As similarly discussed above, a user may undergo sleep treatments that are intended to enhance and improve a user's sleep. Moreover, systems, such as system 200, may include components such as interface 102, processing device 104, and controller 106, which may be coupled to a user, such as user 108.

As shown in Figure 2, components of system 200 may be implemented in a distributed manner. For example, controller 106 may be collocated with user 108 and may be communicatively coupled to processing device 104 via a communications network, such as network 202. In this way, controller 106 may be implemented as a wireless device, such as a wearable device, at user 108, processing device 104 may be implemented remotely in a data processing system, and communications between controller 106 and processing device 104 may be handled via a network 202, which may be the internet. In this way, processing device 104 may be implemented as a personal computer or mobile device located near user 108, or processing device may be implemented as part of a distributed computing platform configured to provide sleep profile enhancement as a Software as a Service (Saas) platform.

Figure 3 illustrates a flow chart of an example of a method for sleep intervention quality estimation, implemented in accordance with some embodiments. As similarly discussed above, a user may undergo sleep treatments and/or interventions that are intended to enhance and improve a user's sleep. As will be discussed in greater detail below, treatment data may be retrieved and analyzed in combination with a user's measured sleep data to generate a sleep model, and such a sleep model may be configured to predict the results of future sleep treatments and/or interventions.

Method 300 may commence with operation 302 during which measurement data may be received from a plurality of data sources. In various embodiments, the measurement data includes measurements of various biological parameters of the user before and after one or more sleep intervention treatments. As discussed above, such measurements may be made via system components, such as electrodes, and may be recorded and stored as measurement data. Moreover, as will be discussed in greater detail below, the raw measurement data may be pre-processed to generate one or more additional measurements, such as biomarkers.

Method 300 may commence with operation 304 during which treatment data may be received. In various embodiments, the treatment data may include one or more data values representing the one or more sleep intervention treatments that were utilized. For example, such treatment data may include stimulation parameters used to apply the stimuli to the user. In another example, the treatment data may include pharmacological data representing doses of pharmacological treatments given to the user. In this way, the treatment data may include data representing various different treatment modalities for a particular user.

Method 300 may commence with operation 306 during which at least one sleep model may be generated for the user. As will be discussed in greater detail below, a sleep model may be generated that is configured to model the user's brain and, more specifically, generate an estimated response of a user's brain to a particular sleep intervention treatment. As will also be discussed in greater detail below, previously collected measurement data and treatment data may be used to construct a sleep model that models the response of the user's brain to such treatments and stimulation parameters. In one example, the measurement and treatment data may be used as training data for various machine learning techniques to construct the sleep model.

Method 300 may commence with operation 308 during sleep intervention quality assessment metrics may be generated. Accordingly, as will be discussed in greater detail below, the sleep model may be configured to receive an input that may be one or more input treatment parameters. Moreover, the model may be configured to generate an output base on the received input. The output may include various metrics that provide an estimation of the user's brain's response to such input treatment parameters. In this way, input treatment parameters may be configured to represent a proposed sleep intervention treatment, and the generated output may include metrics that are configured to represent an estimation of how the user's brain will respond to the proposed sleep intervention treatment.

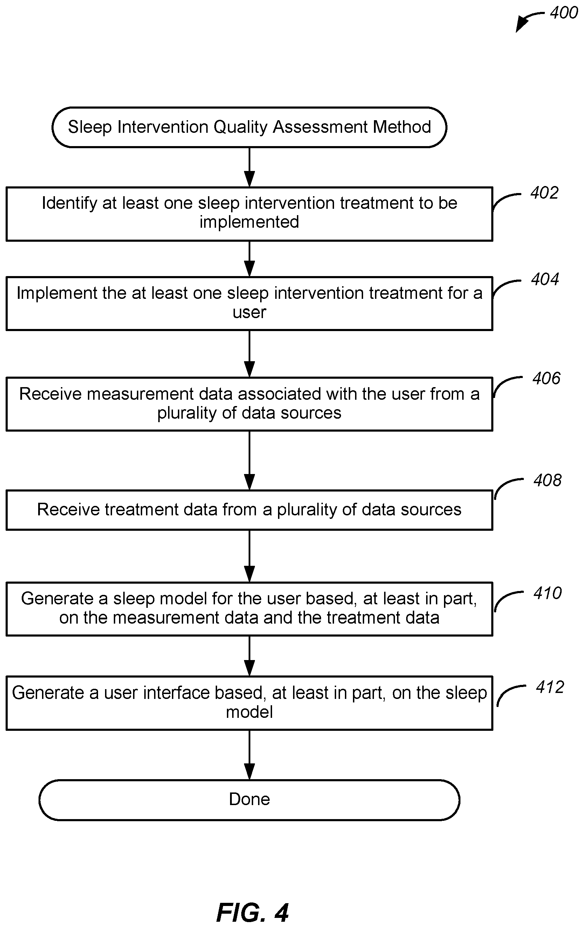

Figure 4 illustrates a flow chart of another example of a method for sleep intervention quality estimation, implemented in accordance with some embodiments. As discussed above, treatment data may be retrieved and analyzed in combination with measurement data to generate a sleep model, and such a sleep model may be configured to predict the results of future sleep treatments and/or interventions. As will be discussed in greater detail below, the sleep model may be constructed using data retrieved from other data sources as well.

Method 400 may commence with operation 402 during at least one sleep intervention treatment may be identified. In various embodiments, the at least one sleep intervention treatment is an initial sleep intervention treatment that will be used to train and generate the sleep model. The at least one sleep intervention treatment may be identified based on one or more treatment parameters that may have been previously specified by an entity, such as a therapist, or may have been entered by another entity such as a user or health care professional based on clinical or therapeutic guidelines.

In some embodiments, the at least one sleep intervention treatment may have already been implemented as part of a previous treatment the user has already undergone. In such a situation, the at least one sleep intervention treatment may be identified based on historical data that is stored for the user. Such historical data may include recorded measurement data for a user, previous treatment data stored for the user, and such historical data may be stored in a user account. Such user account data may be stored and maintained in one or more components of a system such as those discussed above with reference to Figures 1 and 2.

Method 400 may proceed to operation 404 during which the at least one sleep intervention treatment may be implemented. Accordingly, the sleep intervention treatment may be implemented using a system, such as those described above with reference to Figure 1. For example, simulation parameters may be implemented via one or more modalities, such as stimulation via electrodes as well as stimulation using light and sound for visual and aural stimulation. As discussed above, the stimulation parameters may specify a frequency, duration, and timing of the stimulation as the user sleeps.

Method 400 may proceed to operation 406 during which measurement data may be received from a plurality of data sources. As similarly discussed above, as the sleep intervention treatment is applied, measurement data may be collected. In some embodiments the measurement data may be collected via the electrodes. Accordingly, measurement data may include a time course of electrical and neural activity of the user. The measurement data may include other biological measurements as well, such as heart rate and blood pressure measurements. In some embodiments the measurement data may also include self-reported measurements of the intervention treatment efficacy, as well as other measurements of sleep quality such as alertness, relaxation, emotional state, anxiety, insomnia level. These self-report measurements may be after each treatment or could be before and after treatment (with a pre-post difference computation also used). The measurements may be aggregated in a data object and stored as measurement data.

Method 400 may proceed to operation 408 during which treatment data may be received. In various embodiments, the treatment data may include data values that characterize the sleep intervention treatment that was applied, and more specifically, include a detailed representation of the stimulation parameters that were utilized. Accordingly, data values may be retrieved that represent parameters of the stimuli and associated control signals applied during the sleep intervention treatment. In some embodiments, the parameters may also specify the stimulation modality, as well as stimulation parameters each identified modality, such as intensity, frequency, and duration. In some embodiments, such parameters may be stored by the controller and may be retrieved from the controller during operation 408.

Method 400 may proceed to operation 410 during which a sleep model may be generated based, at least in part, on the received measurement data and treatment data. In various embodiments, the sleep model may be generated by using the retrieved measurement data and treatment data as training data for the sleep model. In some embodiments, the measurement data may include direct measurements as well as indirect metrics derived or inferred from the measurements. Accordingly, such measurement data may pre-processed to include the representation of one or more biomarkers of a user. For example, such biomarkers may be parameters of individualized exponential curve fits to the user's spectral data before, during, and after sleep, as well as the different stages of sleep. In some embodiments, biomarkers may include specific power band ratios (for example the delta power over beta power, slow wave power over beta power, or slow wave over low beta power). The biomarkers also include specific resonant frequencies and changes in the resonant frequencies for the user before, during, and after sleep, as well as during the different stages of the sleep. In some embodiments, the biomarkers include absolute or relative power distribution in the different spectral bins/bands for each time period of interest, which may be configured to be specific time windows before and/or after sleep, or specific sleep stages or overall NREM sleep and REM sleep stages.

In various embodiments, the progression of these biomarkers over the user's sleep cycle, starting with pre-sleep, through sleep, and after sleep, may be used to represent and define a sleep profile for the user. Accordingly, the sleep profile may store the values of the biomarkers, as well as difference values identifying changes in such biomarkers. Moreover, the biomarkers and sleep profiles may be aggregated for groups of users, and deviations from the group measurements could be a reported biomarker. Additional biomarkers derived from the measurements may include heart rate based biomarkers like the heart rate (HR) or heart rate variability (HRV) measures that may also be included in an evolving profile that may represent such values before, after, and during the different sleep stages, or as a temporal evolution/time series profile in which measurements are made every few seconds, such as at 30 second intervals. This may also be implemented for movement measurements as well. In some embodiments, the additional measurement data may also include a pre-post differential measure of self-reported survey metrics of alertness, relaxation, anxiety, emotional states. As will be discussed in greater detail below, any of the biomarkers discussed above may be used to generate the quality assessment metrics disclosed herein.

Accordingly, the sleep model may be implemented using one or more machine learning algorithms. More specifically, the sleep models may be developed as functional or phenomenological input-output models that can include machine learning algorithms, such as multi-variate regression, support vector machines, classifiers, deep learning neural networks, hierarchical Bayesian techniques, that are configured to learn the underlying behavior of a user, and model an output of the user based on a received input. In some embodiments, previous treatment measurement data may be used to train the algorithms for a particular user. Once the sleep model has been trained, the sleep model is configured to receive an input identifying proposed treatment parameters, and is further configured to generate an output that provides an estimation of a response of the user based on the received input. Accordingly, inputs to these models may include physiological measurements (such as the electrical activity, heart activity, EOG, movement), self-reported measurements, and the treatment parameters, such as the stimulation modality, and the specific stimulation parameters, such as intensity and frequency. It will be appreciated that the training data and inputs to the model may include the biomarkers discussed above.

Moreover, as also discussed above, such proposed treatment parameters may be stimulation parameters, and the estimation of the user response may be estimated measurement data generated by the sleep model. In some embodiments, the sleep model may include a baseline reference model of the user response generated based on prior measurement data obtained during previous interventions, and the estimates of the user response may be tracked and stored as incremental changes from the user's baseline.

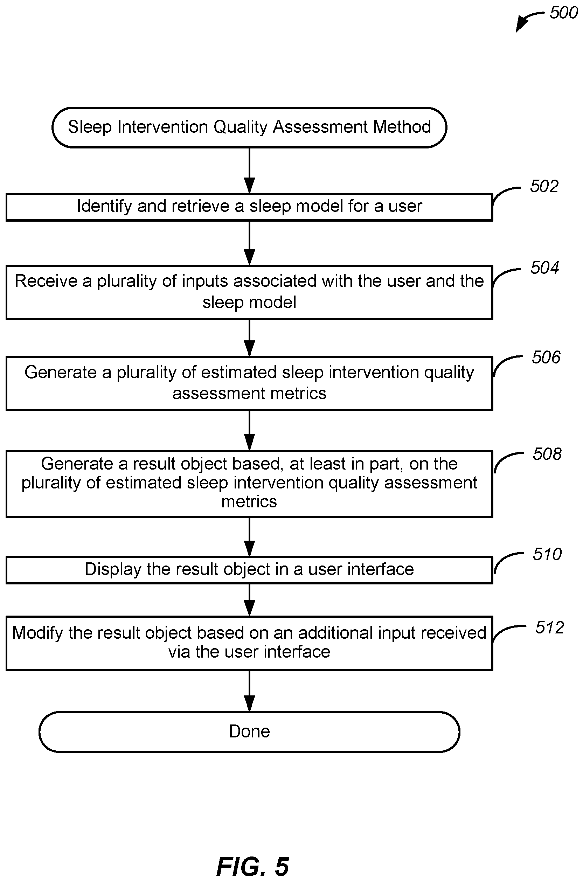

Method 400 may proceed to operation 412 during which a user interface may be generated based, at least in part, on the sleep model. In various embodiments, the user interface is configured to provide a user with outputs generate by the sleep model, and is also configured to receive one or more inputs from the user. Accordingly, the user interface may be a user interface screen displayed on a display device that is configured to receive one or more inputs from the user via one or more input devices, such as a mouse and keyboard, or a touchscreen. Thus, the user interface may be configured to enable a user to input proposed treatment parameters, and be provided with an estimated result of the treatment as well as an assessment of the efficacy of such proposed treatment. For example, the user may be provided with various user interface elements, such as data fields and drop-down menus, that may be used to provide inputs to the processing device discussed above. Additional details of the inputs provided by the user are discussed in greater detail below with reference to Figure 5.

In various embodiments, such user inputs may also be used to modify a presentation of the user screen. Accordingly, the user inputs may be used to customize the results that are displayed in the user interface, and may be used to filter such results based on user preferences. In various embodiments, the user inputs may also be used to implement one or more operations, such as sending a message that includes a report via one or more communications modalities, such as email. Moreover, the user demographic attributes (such as gender, ethnicity, and health condition) can be utilized when generating estimates of changes in the user's response, as well as comparing that against group or aggregated data.

Figure 5 illustrates a flow chart of yet another example of a method for sleep intervention quality estimation, implemented in accordance with some embodiments. As discussed above, a sleep model may be configured to predict the results of future sleep treatments and/or interventions. As will be discussed in greater detail below, various inputs may be received that represent a proposed sleep intervention treatment, and an output may be generated that represents an estimation of how the user will respond to such treatments. As will also be discussed in greater detail below, other estimations of biometrics of the user may be generated, and the receiving of inputs and display of outputs may be implemented via a user interface.

Method 500 may commence with operation 502 during which a sleep model may be identified and retrieved. As discussed above, a sleep model may have been generated for a user based on available measurement and treatment data. Accordingly, during operation 502 the user's sleep model may be identified and retrieved. In various embodiments, the sleep model may be identified and retrieved responsive to an input, such as a function call from an application or an input received via an input of a user interface. For example, a user may provide an input that indicates sleep intervention quality estimation metrics are to be generated.

Method 500 may proceed to operation 504 during which a plurality of inputs may be received. As similarly discussed above, the inputs may be received from a user, and may be received via a user interface. In various embodiments, the inputs include one or more data values that are configured to identify a plurality of proposed treatment parameters. In one example, the inputs may identify particular proposed stimulation parameters. In another example, the inputs may identify a particular type of proposed treatment, and the received input may be pre-processed by a system component, such as a processing device and/or controller, to generate the proposed stimulation parameters. For example, the input may identify a type of stimulation protocol, and the processing device may be configured to generate specific stimulation parameters based on the identified type of stimulation protocol. In one example, the processing device may map the identified type of stimulation protocol to specific stimulation parameters based on a predetermined mapping stored in a data table.

Method 500 may proceed to operation 506 during which a plurality of estimated sleep intervention quality metrics may be generated based, at least in part, on the sleep model and the received plurality of inputs. In various embodiments, the plurality of estimated sleep intervention quality metrics is configured to represent a difference between the generated output of the sleep model and a plurality of ideal sleep parameters for a user. Such ideal sleep parameters may be retrieved from a database. In some embodiments, the ideal sleep parameters may be sleep parameters that represent ideal values of sleep parameters for the user. Such ideal sleep parameters may have been previously generated by, for example, averaging measurement data from a group of healthy users that have similar biographical parameters, such as age and sex, as the user. In some embodiments, the ideal sleep parameters can represent the ideal values of sleep parameters for a group with similar health conditions or parameters as the user.

Method 500 may proceed to operation 508 during which a result object may be generated. In various embodiments, the result object is a data object configured to store outputs of the sleep model. Accordingly, the result object is generated to include the outputs of the sleep model that were generated as well as the plurality of estimated sleep intervention quality metrics.

Method 500 may proceed to operation 510 during which the result object may be displayed in a user interface. Accordingly, one or more aspects of the result object may be displayed to the user. For example, the user may be provided with a graphical representation of the estimated sleep intervention quality metrics that provides the user with a visual representation of how effective the proposed sleep treatment would be. In one example, the representation of the estimated sleep intervention quality metrics may display one or more estimated outputs of the sleep model, may additionally display one or more ideal values associated with the estimated outputs, and may also display one or more visual indicators of differences between the two, such as a difference value and a color coding scheme associated with such difference values and implemented based on a magnitude of the differences.

Method 500 may proceed to operation 512 during which the result object may be modified via an additional input received via the user interface. Accordingly, the user may provide one or more inputs that modifies or customizes the displayed results. For example, the user may filter the results or remove some results from view. Moreover, the user may provide an input that changes the proposed treatment parameters. Accordingly, the user may be provided with the estimated sleep intervention quality metrics, and may update the proposed treatment parameters based on the estimated sleep intervention quality metrics. In this way, the user may be provided with an estimated result of a proposed treatment, and may subsequently implement one or more changes to update the proposed treatment to increase the efficacy of the proposed treatment.

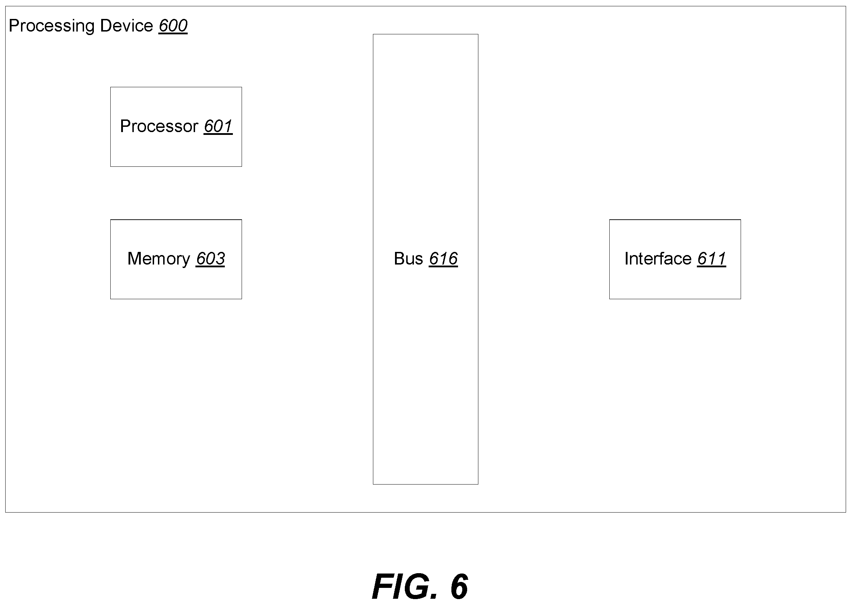

Figure 6 illustrates an example of a processing device that can be used with various embodiments. For instance, the processing device 600 can be used to implement any of processing device 104 and controller 106 according to various embodiments described above. In addition, the processing device 600 shown can be implemented in conjunction with a computing system on a mobile device or on a computer or laptop, etc. According to particular example embodiments, a processing device 600 suitable for implementing particular embodiments of the present invention includes a processor 601, a memory 603, an interface 611, and a bus 616 (e.g., a PCI bus). The interface 611 may include separate input and output interfaces, or may be a unified interface supporting both operations. When acting under the control of appropriate software or firmware, the processor 601 is responsible for tasks such as sleep model computation and generation. Various specially configured devices can also be used in place of a processor 601 or in addition to processor 601. The complete implementation can also be done in custom hardware. The interface 611 may be configured to send and receive data packets or data segments over a network. Particular examples of interfaces the device supports include Ethernet interfaces, frame relay interfaces, cable interfaces, DSL interfaces, token ring interfaces, and the like. In various embodiments, interface 611 may also be a wired connection or a bus with appropriate communications ports.

In addition, various very high-speed interfaces may be provided such as fast Ethernet interfaces, Gigabit Ethernet interfaces, ATM interfaces, HSSI interfaces, POS interfaces, FDDI interfaces and the like. Generally, these interfaces may include ports appropriate for communication with the appropriate media. In some cases, they may also include an independent processor and, in some instances, volatile RAM. The independent processors may control such communications intensive tasks as packet switching, media control and management.

According to particular example embodiments, the processing device 600 uses memory 603 to store data and program instructions and maintain a local side cache. The program instructions may control the operation of an operating system and/or one or more applications, for example. The memory or memories may also be configured to store received metadata and batch requested metadata.

Because such information and program instructions may be employed to implement the systems/methods described herein, the present invention relates to tangible, machine readable media that include program instructions, state information, etc. for performing various operations described herein. Examples of machine-readable media include memory devices such as non-volatile memory devices, volatile memory devices, and may also utilize optical media such as CD-ROM disks and DVDs, and hardware devices that are specially configured to store and perform program instructions, such as read-only memory devices (ROM) and programmable read-only memory devices (PROMs). Examples of program instructions include both machine code, such as produced by a compiler, and files containing higher level code that may be executed by the computer using an interpreter.

While the present disclosure has been particularly shown and described with reference to specific embodiments thereof, it will be understood by those skilled in the art that changes in the form and details of the disclosed embodiments may be made without departing from the spirit or scope of the disclosure. Specifically, there are many alternative ways of implementing the processes, systems, and apparatuses described. It is therefore intended that the invention be interpreted to include all variations and equivalents that fall within the true spirit and scope of the present invention. Moreover, although particular features have been described as part of each example, any combination of these features or additions of other features are intended to be included within the scope of this disclosure. Accordingly, the embodiments described herein are to be considered as illustrative and not restrictive.

## Claims

1. A system comprising:
a communications interface configured to receive measurement data from a plurality of data sources, the measurement data comprising a plurality of measurements of biological parameters of a user before and after a sleep intervention treatment, the communications interface being further configured to receive treatment data comprising one or more treatment parameters associated with the sleep intervention treatment;
a processing device comprising one or more processors configured to generate at least one sleep model for the user based on the received measurement data and treatment data, the processing device being further configured to generate a user interface based, at least in part, on the at least one sleep model; and
a memory device configured to store the at least one sleep model.

2. The system of claim 1 or 2, wherein the user interface is configured to display an output to the user, and receive an input form the user via one or more data fields.

3. The system of claim 1, wherein the processing device is further configured to:
receive a plurality of inputs from the user; and
generate a plurality of estimated sleep intervention quality metrics based, at least in part, on the received plurality of inputs and the at least one sleep model.

4. The system of claim 3, wherein the plurality of estimated sleep intervention quality metrics comprises a representation of a difference between a generated output of the at least one sleep model and a plurality of ideal sleep parameters for the user.

5. The system of claim 3, wherein the processing device is further configured to:
generate a result object based, at least in part, on the plurality of estimated sleep intervention quality metrics.

6. The system of claim 5, wherein the processing device is further configured to:
display the result object in the user interface.

7. The system of claim 6, wherein the processing device is further configured to:
modify the result object based on an additional input received via the user interface.

8. The system of claim 7, wherein the additional input comprises a change to one or more proposed treatment parameters.

9. A device comprising:
a communications interface configured to receive measurement data from a plurality of data sources, the measurement data comprising a plurality of measurements of biological parameters of a user before and after a sleep intervention treatment, the communications interface being further configured to receive treatment data comprising one or more treatment parameters associated with the sleep intervention treatment; and
a processing device comprising one or more processors configured to generate at least one sleep model for the user based on the received measurement data and treatment data, the processing device being further configured to generate a user interface based, at least in part, on the at least one sleep model.

10. The device of claim 9, wherein the user interface is configured to display an output to the user, and receive an input form the user via one or more data fields.

11. The device of claim 9 or 10, wherein the processing device is further configured to:
receive a plurality of inputs from the user; and
generate a plurality of estimated sleep intervention quality metrics based, at least in part, on the received plurality of inputs and the at least one sleep model.

12. The device of claim 11, wherein the plurality of estimated sleep intervention quality metrics comprises a representation of a difference between a generated output of the at least one sleep model and a plurality of ideal sleep parameters for the user.

13. The device of claim 11 or 12, wherein the processing device is further configured to:
generate a result object based, at least in part, on the plurality of estimated sleep intervention quality metrics.

14. The device of claim 13, wherein the processing device is further configured to:
display the result object in the user interface.

15. The device of claim 14, wherein the processing device is further configured to:
modify the result object based on an additional input received via the user interface.
